(19) Europäisches Patentamt / European Patent Office / Office européen des brevets

(11) **EP 4 787 380 A1**

(12) **EUROPEAN PATENT APPLICATION**
published in accordance with Art. 153(4) EPC

(43) Date of publication:
**05.08.2026 Bulletin 2026/32**

(21) Application number: **24872125.0**

(22) Date of filing: **24.09.2024**

(51) International Patent Classification (IPC):
**G16B 40/20** (2019.01)

(52) Cooperative Patent Classification (CPC):
**G16B 40/20**

(86) International application number:
**PCT/JP2024/033802**

(87) International publication number:
**WO 2025/070334 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 JP 2023166428**

(71) Applicant: **The University of Osaka Osaka 565-0871 (JP)**

(72) Inventors:
• **STANDLEY, Daron Michaelangelo**
  **Suita-shi, Osaka 565-0871 (JP)**
• **LI, Songling**
  **Suita-shi, Osaka 565-0871 (JP)**
• **XU, Zichang**
  **Suita-shi, Osaka 565-0871 (JP)**

(74) Representative: **Bandpay & Greuter**
  **11 rue Christophe Colomb**
  **75008 Paris (FR)**

(54) **INFORMATION PROCESSING METHOD, LEARNING MODEL GENERATION METHOD, LEARNING MODEL, COMPUTER PROGRAM, AND INFORMATION PROCESSING DEVICE**

(57) An information processing method, a learning model generation method, a learning model, a computer program, and an information processing apparatus are provided.

An information processing method executed by a computer, the method includes acquiring data of adaptive immune receptors of a subject, executing computation based on the acquired data of adaptive immune receptors of the subject by using a learning model trained to output, on a basis of data of adaptive immune receptors, disease information estimated from the adaptive immune receptors, and outputting the disease information estimated from the adaptive immune receptors of the subject, on a basis of a computation result obtained by execution using the learning model.

*FIG. 4*

| DISEASE NAME | CLASS | DONOR | AIRs | AIR type |
|---|---|---|---|---|
| COVID-19 | DISEASE | 44 | 106,640 | BCR Heavy |
| | HEALTHY | 58 | 174,139 | |
| | TOTAL | 102 | 280,779 | |
| COLORECTAL CANCER (CRC) | DISEASE | 14 | 115,394 | TCR Alpha |
| | HEALTHY | 18 | 148,620 | |
| | TOTAL | 32 | 264,014 | |
| AUTOIMMUNE HEPATITIS (AIH) | DISEASE | 59 | 338,541 | TCR Beta |
| | HEALTHY | 59 | 390,507 | |
| | TOTAL | 118 | 729,048 | |
| PRIMARY BILIARY CHOLANGITIS (PBC) | DISEASE | 30 | 144,298 | TCR Beta |
| | HEALTHY | 59 | 390,507 | |
| | TOTAL | 89 | 534,805 | |
| BRONCHOGENIC CARCINOMA (BC) | DISEASE | 20 | 85,580 | TCR Beta |
| | HEALTHY | 14 | 30,630 | |
| | TOTAL | 34 | 116,210 | |
| HUMAN IMMUNODEFICIENCY VIRUS (HIV) | DISEASE | 94 | 5,202,133 | BCR Heavy |
| | HEALTHY | 128 | 7,934,405 | |
| | TOTAL | 222 | 13,136,538 | |

## Description

[Technical Field]

The present disclosure relates to an information processing method, a learning model generation method, a learning model, a computer program, and an information processing apparatus.

[Background Art]

An adaptive immune system includes T cell receptors and B cell receptors as molecules that specifically recognize a wide variety of pathogens. Since organisms cannot predict pathogens that invade from the outside world, they respond to a wide variety of pathogens by maintaining the diversity of T cell receptors and B cell receptors. The T cell receptor and the B cell receptor are composed of combinations of a plurality of gene regions, and for example, it is estimated that, in humans, there are $10^{15}$ to $10^{20}$ types of T cell receptors and $10^{16}$ to $10^{18}$ types of B cell receptors.

As a method for determining a profile of recombinant DNA sequences in T cells and B cells, Patent Literature 1 discloses a method of collecting a sample derived from a subject including T cells and B cells, spatially isolating individual molecules of genomic DNA from the cells, determining the sequences of the spatially isolated individual molecules, and creating a profile of recombinant DNA sequences.

[Prior Art Document]

[Patent Document]

[Patent Document 1] Japanese Translation of PCT International Application Publication No. 2012-508011

[Summary of Invention]

[Problems to be Solved by Invention]

The method of Patent Literature 1, in which the profile of recombinant DNA sequences is created for individual T cells and B cells, has limitations in terms of generalizability for unknown data.

In one aspect, an object is to provide an information processing method, a learning model generation method, a learning model, a computer program, and an information processing apparatus capable of estimating a disease by using a learning model capable of generalizing even unknown data of adaptive immune receptors.

[Means for Solving Problems]

An information processing method of the present disclosure executed by a computer, the method includes acquiring data of adaptive immune receptors of a subject; executing computation based on the acquired data of adaptive immune receptors of the subject by using a learning model trained to output, on the basis of data of adaptive immune receptors, disease information estimated from the adaptive immune receptors; and outputting the disease information estimated from the adaptive immune receptors of the subject, on the basis of a computation result obtained by execution using the learning model.

[Effects of Invention]

In one aspect, a disease can be estimated by using the learning model capable of generalizing even unknown data of adaptive immune receptors.

[Brief Description of Drawings]

Fig. 1 is an explanatory diagram illustrating an outline of the present disclosure.
Fig. 2 is a block diagram illustrating an internal configuration of an information processing apparatus.
Fig. 3 is a flowchart illustrating a procedure of processing executed by the information processing apparatus in a learning phase.
Fig. 4 is a table showing an example of a data set used by the information processing apparatus in the learning phase.

Fig. 5 is an explanatory diagram illustrating a first classification method of AIR data.

Fig. 6 is an explanatory diagram illustrating a second classification method for AIR data.

Fig. 7 is an explanatory diagram illustrating an outline of a method of calculating cluster frequency CF.

Fig. 8 is an explanatory diagram illustrating an outline of a method of calculating cluster occupancy CO.

Fig. 9 is a flowchart illustrating a procedure of processing executed by the information processing apparatus in an operation phase.

Fig. 10 is a graph showing evaluation results of sparsity.

Fig. 11 is a table showing comparison results of ROC AUC of a test set using four different types of features.

Fig. 12 is a graph showing ROC curves of CCF and PCO.

[Mode for Carrying out Invention]

Hereinafter, the present invention will be described in detail with reference to the drawings illustrating embodiments thereof.

(First embodiment)

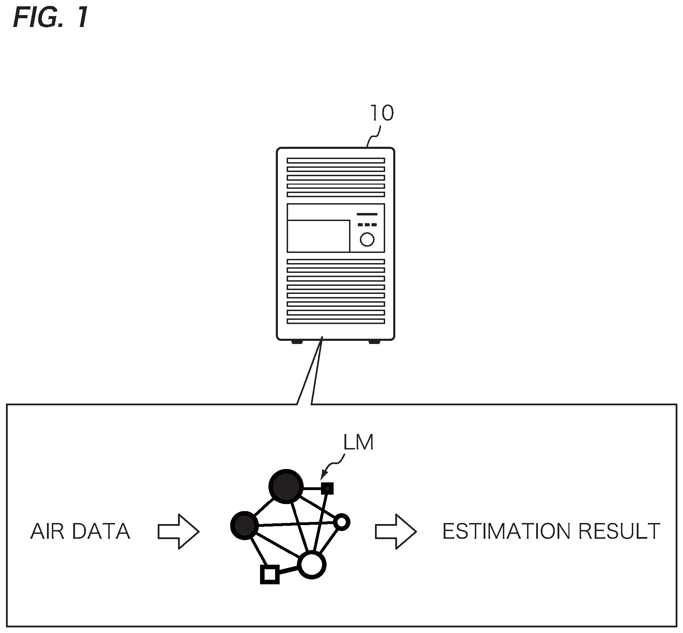

Fig. 1 is an explanatory diagram illustrating an outline of the present disclosure. The present disclosure focuses on biomarker discovery utilizing an adaptive immune receptor (AIR). Hereinafter, the adaptive immune receptor is also referred to as an AIR. In the present embodiment, AIR data and disease data of each donor are collected from a plurality of donors. Donors include patients affected by diseases including, for example, infectious diseases (such as HIV and COVID-19), autoimmune diseases (such as autoimmune hepatitis and primary biliary cholangitis), and cancers (such as breast cancer and colorectal cancer).

In a learning phase, an information processing apparatus 10 according to the present embodiment generates a learning model LM that, in a case where data of AIRs of a subject is input, outputs disease information estimated from the AIRs, by using, as training data, a data set including data of AIRs of a plurality of donors and disease-related data of each donor. In addition, in an operation phase after completion of learning, the information processing apparatus 10 according to the present embodiment acquires AIR data from a new subject, executes computation by the learning model LM on the basis of the acquired AIR data, and outputs disease information estimated by the learning model LM.

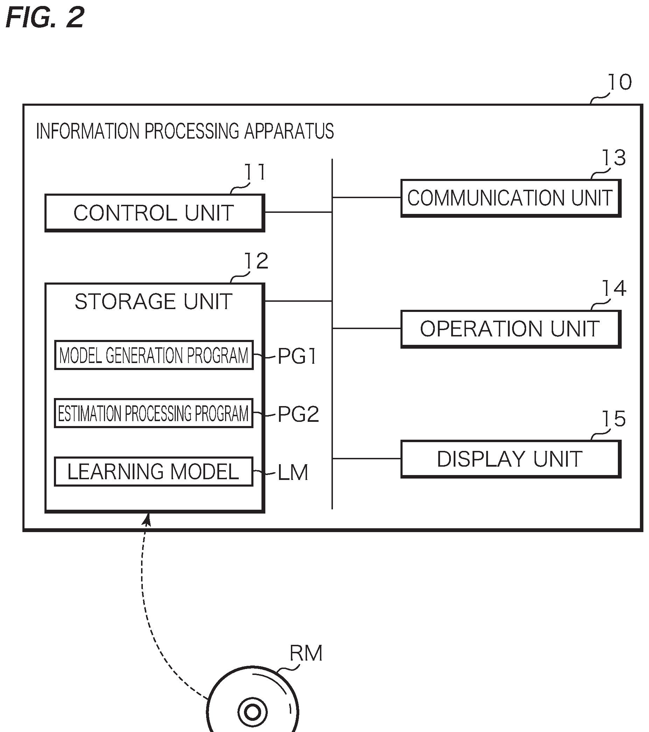

Fig. 2 is a block diagram illustrating an internal configuration of the information processing apparatus 10. The information processing apparatus 10 is a dedicated or general-purpose computer, and includes a control unit 11, a storage unit 12, a communication unit 13, an operation unit 14, and a display unit 15.

The control unit 11 includes, for example, a central processing unit (CPU), a read only memory (ROM), and a random access memory (RAM). The ROM included in the control unit 11 stores a control program and the like for controlling an operation of each unit of hardware included in the information processing apparatus 10. The CPU in the control unit 11 reads and executes a control program stored in the ROM and a computer program to be described later stored in the storage unit 12 to control the operation of each unit of the hardware, thereby causing the entire apparatus to function as the information processing apparatus 10 of the present disclosure. The RAM included in the control unit 11 temporarily stores data utilized during execution of computation and control.

In the embodiment, the control unit 11 includes the CPU, the ROM, and the RAM, but the configuration of the control unit 11 is not limited to the above configuration. The control unit 11 may be, for example, one or more control circuits or arithmetic circuits including a graphics processing unit (GPU), a field programmable gate array (FPGA), a digital signal processor (DSP), a quantum processor, a volatile or nonvolatile memory, or the like. In addition, the control unit 11 may have functions such as a clock that outputs date and time information, a timer that measures an elapsed time from when a measurement start instruction is given to when a measurement end instruction is given, and a counter that counts numbers.

The storage unit 12 includes a storage device such as a hard disk drive (HDD) or a solid state drive (SSD). The storage unit 12 stores various computer programs executed by the control unit 11 and various data utilized by the control unit 11.

The computer program stored in the storage unit 12 includes a model generation program PG1 that causes a computer to execute processing of acquiring a data set including data of adaptive immune receptors obtained for a plurality of donors and disease data of each donor, and using, as training data, the acquired data set to generate a learning model LM that outputs disease information estimated from an AIR when AIR data of a subject is input. In addition, the computer program stored in the storage unit 12 includes an estimation processing program PG2 that acquires AIR data of a subject, inputs the acquired AIR data of the subject to the learning model LM to execute computation, and outputs disease information obtained from the learning model LM.

Each of the computer programs may be a single computer program or the computer programs may be a program group including a plurality of computer programs. In addition, the computer program may partially use an existing library.

Each of the computer programs may be executed by a single computer, or the computer programs may be executed by a plurality of computers in cooperation with each other.

The computer program may be provided by a non-transitory recording medium RM (program product) in which the computer program is readably recorded. The recording medium RM is a portable memory such as a CD-ROM, a USB memory, or a secure digital (SD) card. The control unit 11 reads various computer programs from the recording medium RM by using a reading device (not illustrated), and stores the read various computer programs in the storage unit 12. In addition, the computer program may be provided by communication. The control unit 11 acquires a computer program by communication via the communication unit 13, and stores the acquired computer program in the storage unit 12.

In addition, the storage unit 12 stores a learning model LM trained to output disease information of a subject on the basis of data of adaptive immune receptors of the subject. More specifically, the learning model LM is trained so as to output information on a disease affecting the subject when a feature (such as paratope cluster occupancy described later) extracted from the data of adaptive immune receptors is input. As the learning model LM, a classifier such as eXtreme Gradient Boosting (XGBoost), a Support Vector Machine (SVM), a Bayesian network, a regression tree, or a Light Gradient Boosting Machine (LightGBM) is used. Regarding the trained learning model LM, the storage unit 12 stores information describing the learning model LM and trained parameters such as a layer structure of the model, the number of nodes constituting each layer, a connection relationship, a weighting coefficient and a bias between nodes.

Alternatively, the learning model LM may be stored in an external server accessible from the information processing apparatus 10. In this case, the information processing apparatus 10 may access the external server via communication and perform computation using the learning model LM.

The communication unit 13 includes a communication interface for transmitting and receiving various data to and from an external device. As the communication interface of the communication unit 13, a communication interface conforming to a communication standard such as WiFi (registered trademark), a local area network (LAN), Bluetooth (registered trademark), ZigBee (registered trademark), 3G, 4G, 5G, or long term evolution (LTE) can be used. In a case where data to be transmitted is input from the control unit 11, the communication unit 13 transmits data to a destination external device, and in a case where data transmitted from the external device is received, the communication unit 13 outputs the received data to the control unit 11. In the present embodiment, an example of the external device is a terminal device used by a medical worker such as a doctor or a subject.

The operation unit 14 includes operation devices such as a touch panel, a keyboard, and a switch, and receives various operations and settings by a user or the like. The control unit 11 performs appropriate control on the basis of various operation information given from the operation unit 14, and stores setting information in the storage unit 12 as necessary.

The display unit 15 includes a display device such as a liquid crystal display or an organic electro-luminescence (EL) display, and displays information to be presented, in response to an instruction from the control unit 11.

In the present embodiment, the information processing apparatus 10 may be a single computer or a computer system including a plurality of computers, peripheral devices, and the like. In addition, the information processing apparatus 10 may be a virtual machine having virtualized entity, or may be a cloud.

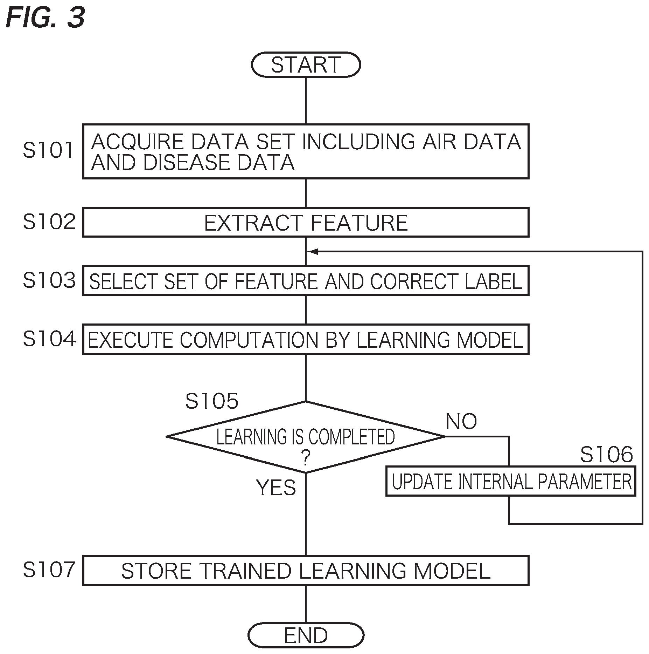

Fig. 3 is a flowchart illustrating a procedure of processing executed by the information processing apparatus 10 in the learning phase. The control unit 11 of the information processing apparatus 10 performs the following processing by executing the model generation program PG1 in the learning phase of generating the learning model LM.

First, the control unit 11 acquires a data set including AIR data obtained for a plurality of donors and disease data of each donor (step S101). A plurality of pieces of AIR data are obtained for each donor. In addition, as the disease data, a correct label indicating whether each donor is affected by any disease such as COVID-19, CRC (colorectal cancer), AIH (autoimmune hepatitis), PBC (primary biliary cholangitis), BC (bronchogenic carcinoma), and HIV (human immunodeficiency virus), or does not suffer from any disease is acquired.

Next, the control unit 11 extracts a feature from the acquired AIR data (step S102). Although a method of calculating the feature will be described in detail later, here, the feature is extracted by clustering AIR data and calculating the occupancy of AIRs within the resulting clusters. In the present embodiment, at the time of clustering, AIR data is clustered by using, as an index, a paratope pseudo sequence obtained by pseudo-concatenating three complementarity-determining regions CDR1, CDR2, and CDR3 included in the AIR. In the present embodiment, as a comparative example, a method of clustering AIR data by using sequences of V gene, J gene, and CDR3 included in the AIR and a method of extracting a feature by calculating the frequency of AIRs within the resulting clusters will be also described.

Next, the control unit 11 selects, from a data set including a feature calculated for each donor and disease data (correct label) of each donor, a set of the feature and the correct label (step S103), inputs the selected feature to the learning model LM, and executes computation by the learning model LM (step S104). It is assumed that an initial value is set to an internal parameter of the learning model LM.

The control unit 11 calculates an error from the correct label in a process of proceeding with the computation by the learning model LM, and determines whether or not the learning is completed, on the basis of the finally obtained error (step S105). If it is determined that the learning is not completed (S105: NO), the control unit 11 updates the internal parameter of the learning model LM (step S106), and returns the processing to step S103 to continue the learning.

If it is determined that the learning is completed (S105: YES), the trained learning model LM is obtained, and thus, the control unit 11 stores the trained learning model LM in the storage unit 12 (step S107).

Hereinafter, details of processing in the learning phase will be described.

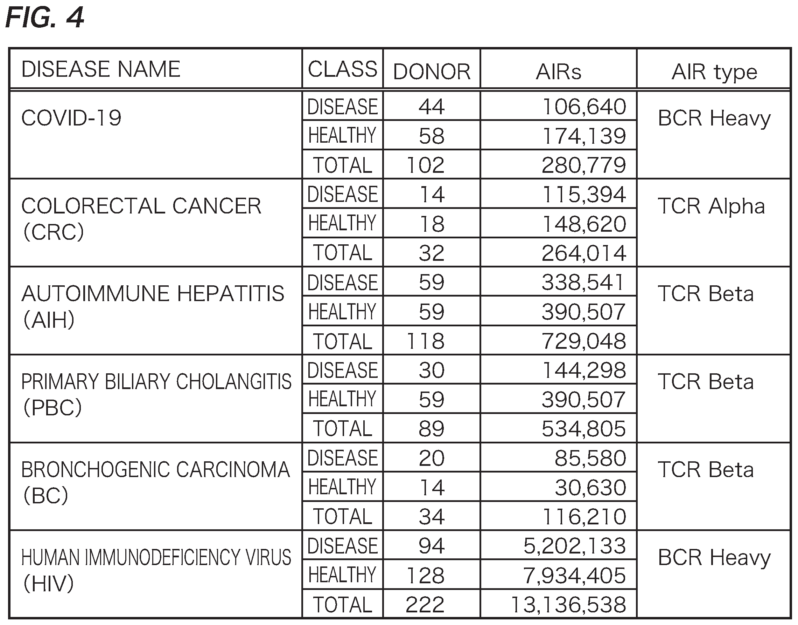

Fig. 4 is a table showing an example of a data set used by the information processing apparatus 10 in the learning phase. In the present embodiment, a data set including 106640 pieces of AIR data from 44 donors affected by COVID-19 and 174139 pieces of AIR data from 58 donors not affected by COVID-19 was used. Similarly, as shown in Fig. 4, a data set including AIR data acquired from donors affected/not affected by CRC (colorectal cancer), AIH (autoimmune hepatitis), PBC (primary biliary cholangitis), BC (bronchogenic carcinoma), HIV (human immunodeficiency virus) was used. These AIR data include BCR (B cell receptor) and TCR (T cell receptor) data. The data set used in the learning phase may be collected in advance and already published as a benchmark data set.

In the example of Fig. 4, a data set in which the AIR data is classified into a class belonging to "disease" and a class belonging to "healthy" is shown for each type of disease, but if the disease has a subtype, a data set in which the AIR data is classified by the subtype may be used. In addition, instead of the data set shown in Fig. 4, a data set in which AIR data is classified by disease severity may be used, or a data set in which AIR data is classified by diseases with a high likelihood of future onset may be used.

In the present embodiment, an XGBoost algorithm was adopted for a binary classification task. Learning was performed with 70% of donors selected at random and all parameters were set to default values, except for positive class weighting based on a ratio between true training donors and false training donors.

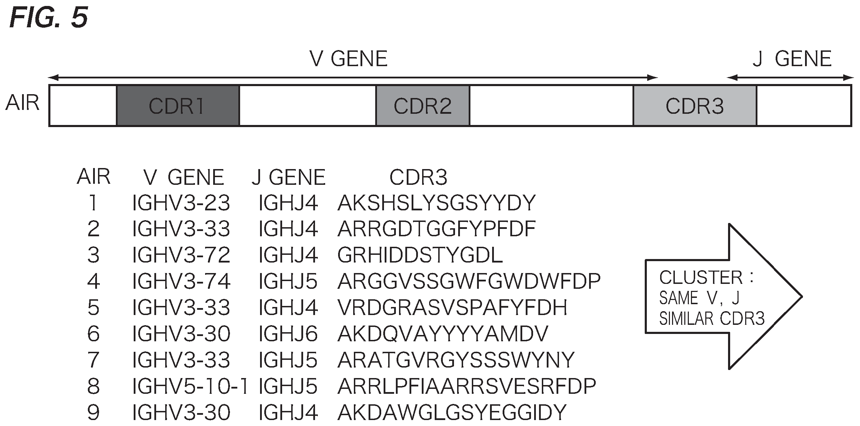

AIR data can be classified according to its AIR representation. Fig. 5 is an explanatory diagram illustrating a first classification method for AIR data. An AIR consists of the amino acid sequence of a B cell receptor or a T cell receptor, and is described by a V gene name, a J gene name, and the position of complementarity-determining region (CDR) within the amino acid sequence. The complementarity-determining region includes three regions (CDR1, CDR2, CDR3). In the first classification method, AIR data is represented by the V gene name, the J gene name, and a CDR3 sequence. A representation using the V gene name, the J gene name, and the CDR3 sequence is referred to as a clonotype representation. In addition, a cluster of AIRs obtained by clustering based on the viewpoint of similarity of CDR3 sequence, while having the same V gene and J gene, is referred to as a clonotype cluster in the present specification.

To cluster in the clonotype representation, AIR data with identical lengths of V gene, J gene, CDR3 were combined. Next, AIR data within these groups were clustered by CDR3 amino acid sequences. In the present embodiment, 80% sequence identity and 100% alignment coverage as implemented in CD-HIT (Fu, L., B. Niu, Z. Zhu, S. Wu and W. Li (2012). "CD-HIT: accelerated for clustering the next-generation sequencing data" Bioinformatics 28 (23): 3150-3152) were used.

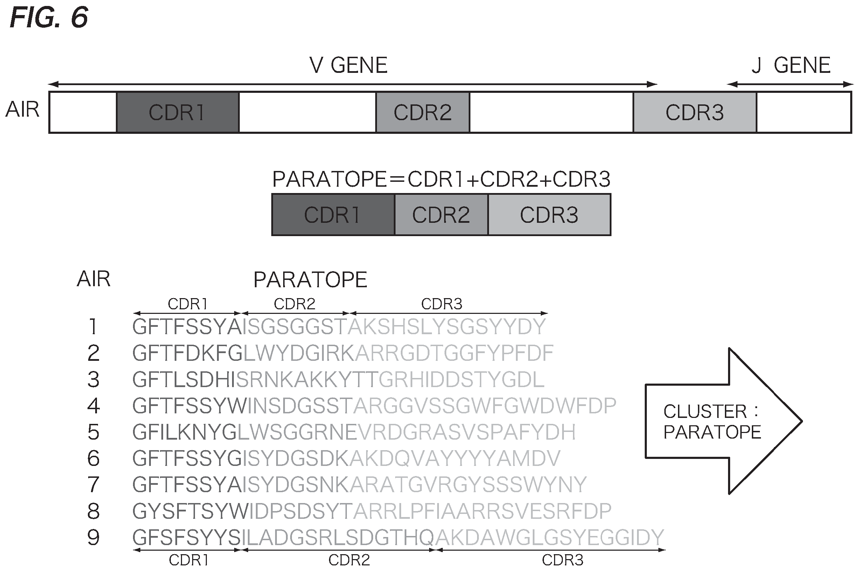

Fig. 6 is an explanatory diagram illustrating a second classification method for AIR data. In the second classification method, AIR data is represented by CDR sequences. A representation using CDR sequences is referred to as a paratope representation. In the present embodiment, one pseudo sequence was constructed by pseudo-concatenating three segments of CDR1, CDR2, and CDR3. A cluster of AIRs obtained by clustering based on the viewpoint of similarity of the pseudo sequence (hereinafter, referred to as a paratope pseudo sequence) is referred to as a paratope cluster in the present specification.

The clustering of the paratope pseudo sequence was performed with a sequence identity threshold of 0.8 and a coverage threshold of 0.9 by using an MMseqs2 search method. Alignment of paratope pseudo sequences with fixed sequence identity and coverage is statistically more significant than CDR3 alignment under identical criteria, due to the long sequence length.

In the present embodiment, an AIR adjacency matrix is constructed from the paratope pseudo sequence. The AIR adjacency matrix can be calculated from the paratope pseudo sequence by using the MMseqs2 search method, for example, with a sequence identity threshold of 0.8 and a coverage threshold of 0.9. That is, assuming that the sequence identity is $p_{ij}$ and the coverage is $q_{ij}$, for a given pair of AIRs $a_i$ and $a_j$, an AIR adjacency matrix $A_{ij}$ is expressed by following Mathematical formula 1.

[Mathematical formula 1]

$$A_{ij} = \begin{cases} 1 \ if \ p_{ij} > 0.8 \ and \ q_{ij} > 0.9 \\ 0 \qquad\qquad\qquad otherwise \end{cases} \quad where \ i \in [1, M] \ and \ k \in [1, M].$$

In the present embodiment, two types of features of the cluster frequency CF and cluster occupancy CO are introduced as features that characterize the AIR data of each donor.

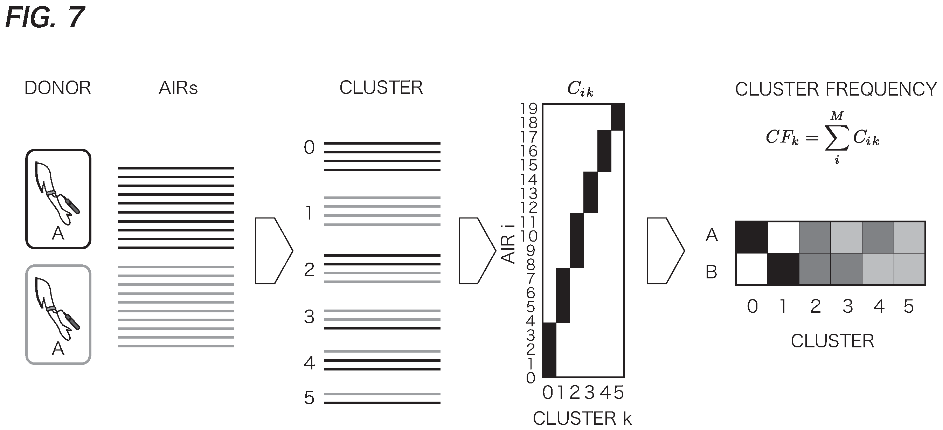

Fig. 7 is an explanatory diagram illustrating an outline of a method of calculating the cluster frequency CF. In the present embodiment, to construct a feature vector for each donor, all AIR data of a given data set were clustered, and each donor was described by the frequency of AIRs within the resulting clusters. Assume that there are M AIRs in a donor D, each belonging to one of N clusters defined as $\{S_1,..., S_N\}$. Formally, a set of different AIRs of the donor D can be defined as $AIR_D = \{a_l,..., a_M\}_D$. Here,

1. for all $a_i \in AIR_D$, there exists $k \in [1, N]$ such that $a_i \in S_k$, and
2. for all $a_i, a_j \in AIR_D$, $a_i = a_j$ only if $i = j$.

Assuming that $C^{M \times N}$ is an $M \times N$ matrix including the one-hot encoding of the cluster assignment of each AIR, each element $C_{ik} \in C^{M \times N}$ is defined as follows.

[Mathematical formula 2]

$$C_{ik} = \begin{cases} 1 & if\ a_i \in S_{k,} \\ 0 & otherwise \end{cases} \quad where\ i \in [1, M]\ and\ k \in [1, N].$$

That is, in a case where $AIRa_i$ belongs to a cluster $S_k$, $C_{ik}$ is equal to 1, and 0 for all other values of k. The cluster frequency CF of a given cluster $S_k$ is calculated by summing over all rows of $C^{M \times N}$ as shown in Mathematical formula 3.

[Mathematical formula 3]

$$CF_k = \sum_i^M C_{ik} \quad where\ k \in [1, N]$$

In the present embodiment, the cluster frequency CF is utilized as a vector representation of each donor. The feature vector of a certain donor D is denoted as $\{CF_1,..., CF_N\}_D$. Hereinafter, a feature based on the clonotype cluster is referred to as clonotype cluster frequency (CCF), and a feature based on the paratope cluster is referred to as paratope cluster frequency (PCF).

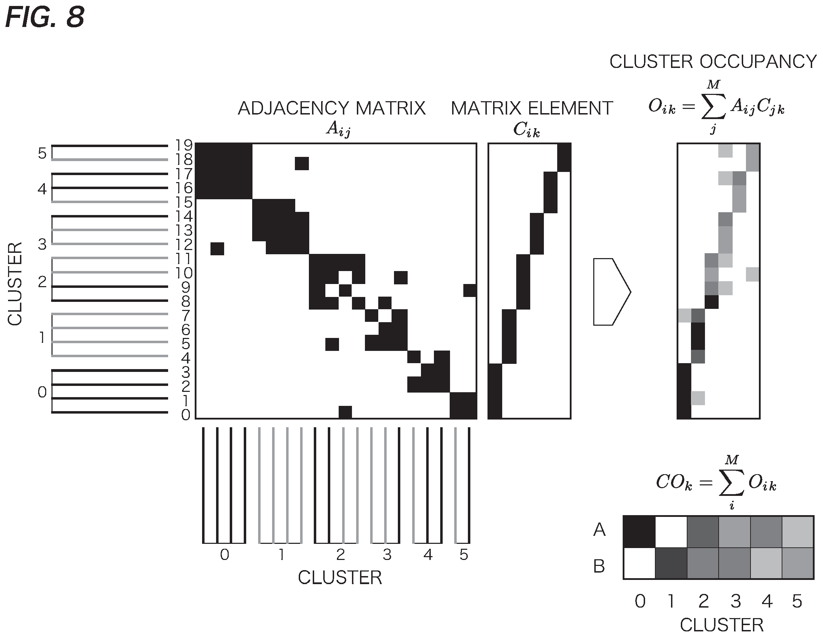

Fig. 8 is an explanatory diagram illustrating an outline of a method of calculating the cluster occupancy CO. Since the distribution of AIR sequences is a long tail distribution, a cluster size also follows the long tail distribution. Furthermore, since the one-hot encoding is used in defining the matrix element $C_{ik}$, the feature becomes very sparse. In this regard, the inventors of the present application assumed that reducing sparsity is effective for downsizing the feature, and utilized an adjacency matrix to identify that AIR belongs to two or more clusters. An occupancy matrix $O^{M \times N}$ is defined by multiplication of an adjacency matrix $A^{M \times M}$ and a cluster matrix $C^{M \times N}$ including one-hot cluster encoding. $\times A^{M \times M}$ is an $M \times M$ symmetric matrix. Formally, it is described by $O^{M \times N} = A^{M \times M} C^{M \times N}$. Here, each element $O_{ik} \in O^{M \times N}$ is defined as follows.

[Mathematical formula 4]

$$O_{ik} = \sum_j^M A_{ij}\ C_{jk}$$

Here, $A_{ij}$ represents similarity between the i-th AIR and the j-th AIR. When clusters having different adjacencies are connected, $O^{M \times N}$ is sparser than $C^{M \times N}$. Furthermore, as in Mathematical formula 3, overall cluster occupancy $CO_k$ of the cluster $S_k$ of a given donor can be calculated by summing the AIRs of all donors.

[Mathematical formula 5]

$$CO_k = \sum_i^M O_{ik}$$

Hereinafter, the cluster occupancy derived from the clonotype cluster is referred to as clonotype cluster occupancy (CCO), and the cluster occupancy derived from the paratope cluster is referred to as paratope cluster occupancy (PCO).

When all pieces of cluster information are used to create the feature vector, the feature vector often has a very high-dimensional feature space. This is mainly because the cluster size follows the long tail distribution, so that a large number of clusters are generated with a very small number of data points. Furthermore, when there are a large number of AIRs, it becomes difficult to calculate a complete matrix. In order to solve these problems, the inventors of the present application propose an algorithm that reduces the number of clusters and also reduces the number of AIRs. Furthermore, by reducing the number of clusters, it is possible to utilize meaningful clusters for feature generation. In order to find meaningful clusters, clusters that meet given population criteria are identified as described below.

First, a set of binary classes is defined as $Y = \{y_1, y_2\}$. Here, $y_1$ represents a class of healthy donors and $y_2$ represents a class of donors with disease. The number of AIRs belonging to the donor of the class $y_p$ in the cluster $S_k$ is defined as $n_p(S_k)$, and the cluster ratio tuple of the cluster $S_k$ is defined as $(r_k^{y_1}, r_k^{y_2})$. Here, $r_k^{y_p}$ is expressed as follows.

[Mathematical formula 6]

$$r_k^{y_p} = \frac{n_p(S_k)}{\sum_{k=1}^{N} n_p(S_k)}$$

That is, $r_k^{y_p}$ represents the ratio of the number of AIRs belonging to the class $y_p$ in the cluster $S_k$ to the total number of AIRs belonging to the class $y_p$.

Next, a threshold parameter $d_{min}$ is introduced. This threshold parameter $d_{min}$ defines a minimum relative population parameter, below which all clusters are deleted and the respective AIRs are ignored. For a class to be selected, at least one value of the cluster ratio tuple is required to be greater than or equal to $d_{min}$. Specifically, if $\max(r_k^{y_1}, r_k^{y_2}) < d_{min}$ with respect to the cluster $S_k$, the cluster $S_k$ is deleted. This downsizing is applied to all data sets in training using $d_{min}$ as a hyperparameter. For very large-scale data sets (for example, the HIV data set shown in Fig. 4), this preprocessing is applied since calculating adjacencies across the entire data set takes a significant amount of time.

For example, in the HIV data set shown in Fig. 4, a total of 13136538 pieces of AIR data are obtained. When the threshold parameter $d_{min}$ was set to 0.05 for the cluster obtained from this AIR data and downsizing was performed as preprocessing, the number of AIRs could be reduced to 4424328. When the value of $d_{min}$ was increased, the number of AIRs could be further reduced, but all AIRs of one or more donors were deleted. The reduced HIV data set was used in all subsequent calculations.

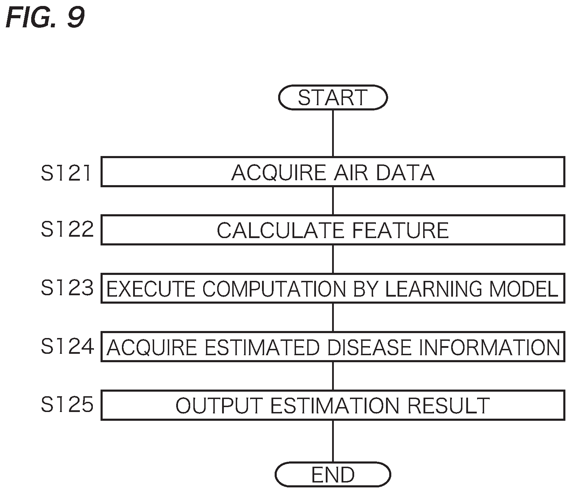

Fig. 9 is a flowchart illustrating a procedure of processing executed by the information processing apparatus 10 in the operation phase. The control unit 11 of the information processing apparatus 10 performs the following processing by executing the estimation processing program PG2 in the operation phase after generating the trained learning model LM.

The control unit 11 acquires AIR data obtained for the subject (step S121), and calculates a feature from the acquired AIR data (step S122). A feature calculation method is similar to the calculation method in the learning phase.

The control unit 11 inputs the feature calculated in step S122 to the trained learning model LM, and executes computation by the learning model LM (step S123). The control unit 11 acquires disease information estimated from the AIR data as a computation result by the learning model LM (step S124).

The control unit 11 outputs the estimation result of step S123 (step S125). The control unit 11 may display the estimation result on the display unit 15, or may notify a terminal of the doctor or the subject through the communication unit 13.

For example, in the data set of Fig. 4, since the AIR data is classified into a class belonging to "disease" and a class belonging to "healthy" for each type of disease, information on whether or not the subject is affected by each disease (presence or absence of disease) is obtained for each type of disease from the learning model LM trained using such a data set. In addition, the control unit 11 may calculate a probability of being affected by each disease in step S123 and output information of the calculated probability as the estimation result. Furthermore, the control unit 11 may output information regarding two or more types of diseases (presence or absence of disease, probability, or the like).

In addition, the learning model LM used in the operation phase may be a learning model trained by using a data set in which AIR data is classified into classes for each disease subtype. In this case, information regarding the disease subtype is obtained as a computation result by the learning model LM. The control unit 11 may output information regarding the disease subtype obtained from the learning model LM.

Furthermore, the learning model LM used in the operation phase may be a learning model trained using a data set obtained by classifying AIR data into classes according to disease severity. In this case, information regarding the disease severity is obtained as the computation result by the learning model LM. The control unit 11 may output the information regarding the disease severity obtained from the learning model LM.

Furthermore, the learning model LM used in the operation phase may be a learning model trained using a data set obtained by classifying AIR data according to diseases with a high likelihood of future onset. In this case, information regarding diseases with a high likelihood of future onset is obtained as the computation result by the learning model LM. The control unit 11 may output information regarding a diseases with a high likelihood of future onset, which is obtained from the learning model LM.

Hereinafter, the evaluation of the learning model LM will be described. Classification performance was evaluated in ROC AUC (receiver operating characteristic curve) using AIR data collected from the remaining 30% donors. In the present embodiment, four workflows having different clustering methods (clonotype or paratope) and feature construction methods (cluster frequency or cluster occupancy) were constructed, and evaluated for each workflow.

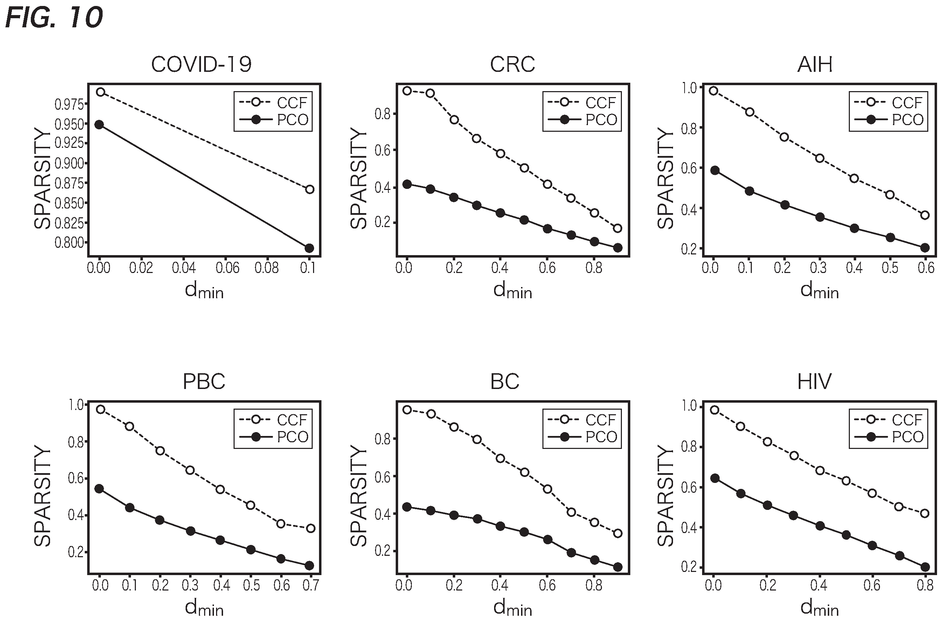

Fig. 10 is a graph showing evaluation results of sparsity. Fig. 10 shows a graph evaluating sparsity for CCF and PCO using AIR data for COVID-19, CRC, AIH, PBC, BC, HIV. A horizontal axis of each graph represents the threshold parameter $d_{min}$, and a vertical axis represents sparsity. The sparsity can be defined as a fraction of zeros in a feature matrix (that is, a set of all feature vectors for a given set of donors). Fig. 10 shows that at all $d_{min}$ values, the PCO feature exhibits lower sparsity than the CCF feature. These results were independent of the clustering method, the sparsity of the PCF features was comparable to the sparsity of the CCF features, and the sparsity of the CCO features was comparable to the sparsity of the PCO features. This evaluation result suggests that a main factor reducing sparsity is the use of paratope adjacency in the calculation of CO features.

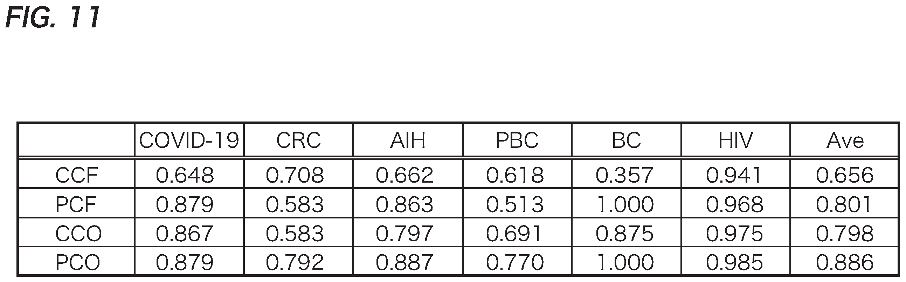

Next, the ROC AUC of test data obtained from data sets for six diseases is shown. Fig. 11 is a table showing comparison results of ROC AUC of a test set using four different types of features. Fig. 11 shows the results of calculating the ROC AUC when using the CCF feature, the PCF feature, the CCO feature, and the PCO feature, respectively, for six diseases of COVID-19, CRC, AIH, PBC, BC, and HIV. Fig. 11 also shows evaluation results for Ave (average) for reference.

In CCF features, it can be seen that generalization could be performed for subjects affected by HIV among the six diseases of COVID-19, CRC, AIH, PBC, BC, and HIV, but generalization could not be performed for new subjects in the remaining five diseases. The use of PCF features showed relatively favorable results in three diseases (COVID-19, AIH, BC) among the five diseases that failed generalization with CCF features and showed poor results in the other two diseases (CRC, PBC). Similarly, the use of CCO features showed favorable results in the same three diseases (COVID-19, AIH, BC), but was poor in other diseases (CRC, PBC). In contrast, the use of PCO features yielded favorable results on all disease data sets.

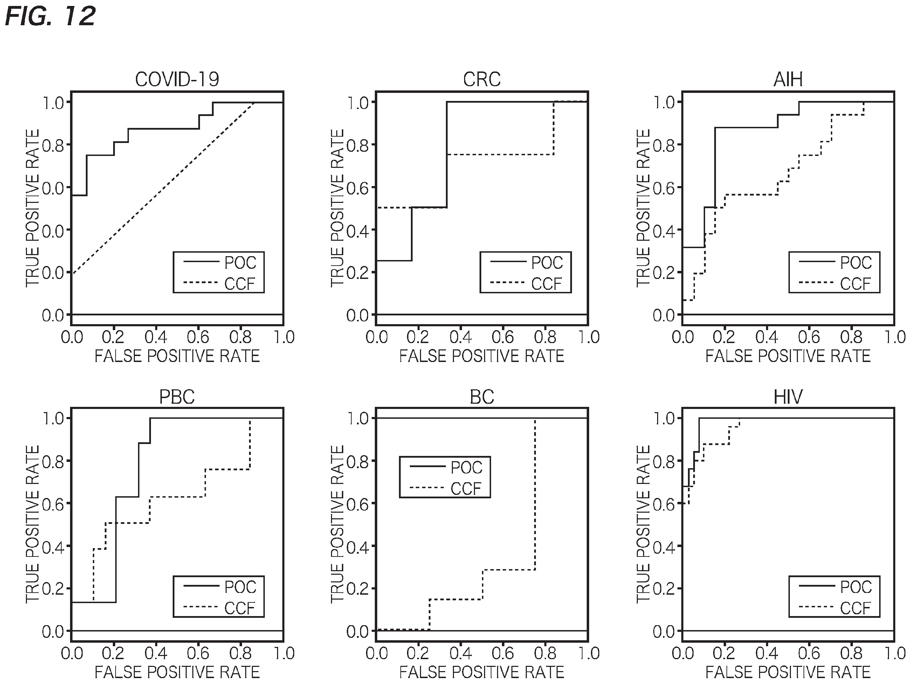

Fig. 12 is a graph showing ROC curves of CCF and PCO. A horizontal axis of each graph represents a false positive rate, and a vertical axis represents a true positive rate (sensitivity). Fig. 12 shows that in all different diseases, the benefit obtained is above the loss. Although the learning model LM does not achieve perfect disease classification, the benchmark indicates that generalizability of features could be fairly tested within the context of the data used in the present embodiment.

As described above, in the present embodiment, by using the learning model LM which is trained by using, as training data, a data set including data of adaptive immune receptors obtained for a plurality of donors and disease-related data of each donor, it has become possible to estimate a disease affecting a subject on the basis of data of adaptive immune receptors of the subject.

Of particular note is that the utilization of the AIR paratope representation has been demonstrated to be beneficial not only for BCR data but also for TCR data. This observation may be attributed to the sparsity of hard cluster encoding features, which was addressed by using the adjacency matrix to transform the cluster frequency into the cluster occupancy. The inclusion of paratope features representing triplets of CDR sequence fragments means the possibility of the presence of a highly clustered network of similarity relationships. The formulation of cluster occupancy effectively captures these relationships, enabling flexible connections of other clusters to an AIR while adjusting strict cluster identity. The improved generalizability of the PCO classification method demonstrates the robustness and effectiveness of this approach.

The embodiment disclosed at present is construed as illustrative in all respects and not restrictive. The scope of the present invention is defined not by the above meaning but by the claims, and is intended to include all modifications within the meaning and scope equivalent to the claims.

[Description of Reference Numerals]

    10      information processing apparatus
    11      control unit
    12      storage unit
    13      communication unit
    14      operation unit

| 15 | display unit |
| PG1 | model generation program |
| PG2 | estimation processing program |
| LM | learning model |

**Claims**

1.  An information processing method executed by a computer, the method comprising:

    acquiring data of adaptive immune receptors of a subject;
    executing computation based on the acquired data of adaptive immune receptors of the subject by using a learning model trained to output, on a basis of data of adaptive immune receptors, disease information estimated from the adaptive immune receptors; and
    outputting the disease information estimated from the adaptive immune receptors of the subject, on a basis of a computation result obtained by execution using the learning model.

2.  The information processing method according to claim 1, wherein the disease information includes at least one of whether the subject is affected by the disease, a disease subtype, disease severity, or information regarding a disease with a high likelihood of future onset.

3.  The information processing method according to claim 1, wherein the disease information includes information on a probability of being affected by the disease.

4.  The information processing method according to claim 1, wherein the disease information includes information regarding two or more types of diseases.

5.  The information processing method according to claim 1, wherein the learning model is a trained learning model which is trained, by using, as training data, a data set including data of adaptive immune receptors obtained for a plurality of donors and disease-related data of each donor, to output, on a basis of data of adaptive immune receptors of a subject, disease information estimated from the adaptive immune receptors.

6.  The information processing method according to claim 5, wherein

    the learning model is a trained learning model in which a relationship between a feature extracted from data of adaptive immune receptors included in the data set and disease-related data included in the data set is learned, and
    the feature is a feature that characterizes a paratope cluster obtained when the data of adaptive immune receptors is clustered by using, as an index, a paratope pseudo sequence configured by pseudo-concatenating segments representing three complementarity-determining regions included in the adaptive immune receptor.

7.  A learning model generation method executed by a computer, the method comprising:

    acquiring a data set including data of adaptive immune receptors obtained for a plurality of donors and disease-related data of each donor; and
    generating, by using the acquired data set as training data, a learning model that outputs, on a basis of data of adaptive immune receptors of a subject, disease information estimated from the adaptive immune receptors.

8.  The learning model generation method according to claim 7 executed by the computer, the method comprising:

    extracting a feature from the data of adaptive immune receptors included in the data set; and
    generating the learning model by learning a relationship between the feature extracted from the data and the disease-related data included in the data set.

9.  The learning model generation method according to claim 8, wherein the feature is cluster occupancy derived from a paratope cluster.

10. The learning model generation method according to claim 8, wherein the feature is a feature that characterizes a paratope cluster generated by clustering the data of adaptive immune receptors with a plurality of types of paratope pseudo sequences.

**11.** The learning model generation method according to claim 10, wherein the paratope pseudo sequence is configured by pseudo-concatenating segments representing three complementarity-determining regions included in the adaptive immune receptor.

**12.** The learning model generation method according to claim 9 executed by the computer, the method comprising:

selecting paratope clusters that meet given population criteria among all paratope clusters; and
extracting the feature by using the selected paratope cluster.

**13.** The learning model generation method according to any one of claims 9 to 12 executed by the computer, the method comprising:
deriving cluster occupancy in the paratope cluster by using an adjacency matrix constructed from similarity of the adaptive immune receptors included in the data.

**14.** A learning model which is trained by using, as training data, a data set including data of adaptive immune receptors obtained for a plurality of donors and disease-related data of each donor, causing a computer to function to:
in a case where data of adaptive immune receptors of a subject is input, execute computation based on the input data of adaptive immune receptors of the subject and output disease information estimated from the adaptive immune receptors of the subject.

**15.** A computer program causing a computer to execute processing of:

acquiring a data set including data of adaptive immune receptors obtained for a plurality of donors and disease-related data of each donor; and
generating, by using the acquired data set as training data, a learning model that outputs, on a basis of data of adaptive immune receptors of a subject, disease information estimated from the adaptive immune receptors.

**16.** A computer program causing a computer to execute processing of:

acquiring data of adaptive immune receptors of a subject;
executing computation based on the acquired data of adaptive immune receptors of the subject by using a learning model trained to output, on a basis of data of adaptive immune receptors, disease information estimated from the adaptive immune receptors; and
outputting the disease information obtained by the learning model.

**17.** An information processing apparatus comprising:

at least one processor, wherein
the processor

acquires data of adaptive immune receptors of a subject;
executes computation based on the acquired data of adaptive immune receptors of the subject by using a learning model trained to output, on a basis of data of adaptive immune receptors, disease information estimated from the adaptive immune receptors; and
outputs the disease information obtained by the learning model.

*FIG. 1*

AIR DATA ⇨ ESTIMATION RESULT

# FIG. 2

INFORMATION PROCESSING APPARATUS — 10

CONTROL UNIT — 11

COMMUNICATION UNIT — 13

STORAGE UNIT — 12

MODEL GENERATION PROGRAM — PG1

ESTIMATION PROCESSING PROGRAM — PG2

LEARNING MODEL — LM

OPERATION UNIT — 14

DISPLAY UNIT — 15

RM

## FIG. 3

```
                          ( START )
                              |
S101  | ACQUIRE DATA SET INCLUDING AIR DATA
      | AND DISEASE DATA
                              |
S102  | EXTRACT FEATURE
                              |
S103  | SELECT SET OF FEATURE AND CORRECT LABEL  <--+
                              |                     |
S104  | EXECUTE COMPUTATION BY LEARNING MODEL      |
                              |                     |
S105                          |              NO     |
             < LEARNING IS COMPLETED > ------------- |
                       ?                      S106  |
                     YES          | UPDATE INTERNAL PARAMETER |
                      |
S107  | STORE TRAINED LEARNING MODEL
                      |
                  ( END )
```

**FIG. 4**

| DISEASE NAME | CLASS | DONOR | AIRs | AIR type |
|---|---|---|---|---|
| COVID-19 | DISEASE | 44 | 106,640 | BCR Heavy |
| | HEALTHY | 58 | 174,139 | |
| | TOTAL | 102 | 280,779 | |
| COLORECTAL CANCER (CRC) | DISEASE | 14 | 115,394 | TCR Alpha |
| | HEALTHY | 18 | 148,620 | |
| | TOTAL | 32 | 264,014 | |
| AUTOIMMUNE HEPATITIS (AIH) | DISEASE | 59 | 338,541 | TCR Beta |
| | HEALTHY | 59 | 390,507 | |
| | TOTAL | 118 | 729,048 | |
| PRIMARY BILIARY CHOLANGITIS (PBC) | DISEASE | 30 | 144,298 | TCR Beta |
| | HEALTHY | 59 | 390,507 | |
| | TOTAL | 89 | 534,805 | |
| BRONCHOGENIC CARCINOMA (BC) | DISEASE | 20 | 85,580 | TCR Beta |
| | HEALTHY | 14 | 30,630 | |
| | TOTAL | 34 | 116,210 | |
| HUMAN IMMUNODEFICIENCY VIRUS (HIV) | DISEASE | 94 | 5,202,133 | BCR Heavy |
| | HEALTHY | 128 | 7,934,405 | |
| | TOTAL | 222 | 13,136,538 | |

# FIG. 5

| | V GENE | | | J GENE |

AIR

| | CDR1 | | CDR2 | | CDR3 | |

| AIR | V GENE | J GENE | CDR3 |
| --- | --- | --- | --- |
| 1 | IGHV3-23 | IGHJ4 | AKSHSLYSGSYYDY |
| 2 | IGHV3-33 | IGHJ4 | ARRGDTGGFYPFDF |
| 3 | IGHV3-72 | IGHJ4 | GRHIDDSTYGDL |
| 4 | IGHV3-74 | IGHJ5 | ARGGVSSGWFGWDWFDP |
| 5 | IGHV3-33 | IGHJ4 | VRDGRASVSPAFYFDH |
| 6 | IGHV3-30 | IGHJ6 | AKDQVAYYYYAMDV |
| 7 | IGHV3-33 | IGHJ5 | ARATGVRGYSSSWYNY |
| 8 | IGHV5-10-1 | IGHJ5 | ARRLPFIAARRSVESRFDP |
| 9 | IGHV3-30 | IGHJ4 | AKDAWGLGSYEGGIDY |

CLUSTER :
SAME V, J
SIMILAR CDR3

# FIG. 6

EP 4 787 380 A1

**FIG. 7**

CLUSTER FREQUENCY

$$CF_k = \sum_i^M C_{ik}$$

FIG. 8

ADJACENCY MATRIX $A_{ij}$

MATRIX ELEMENT $C_{ik}$

CLUSTER OCCUPANCY $O_{ik} = \sum_{j}^{M} A_{ij} C_{jk}$

$CO_k = \sum_{i}^{M} O_{ik}$

CLUSTER

FIG. 9

```
                          ( START )
                              |
S121  |       ACQUIRE AIR DATA               |
                              |
S122  |       CALCULATE FEATURE              |
                              |
S123  | EXECUTE COMPUTATION BY LEARNING MODEL |
                              |
S124  | ACQUIRE ESTIMATED DISEASE INFORMATION |
                              |
S125  |      OUTPUT ESTIMATION RESULT         |
                              |
                          ( END )
```

FIG. 10

FIG. 11

|      | COVID-19 | CRC   | AIH   | PBC   | BC    | HIV   | Ave   |
|------|----------|-------|-------|-------|-------|-------|-------|
| CCF  | 0.648    | 0.708 | 0.662 | 0.618 | 0.357 | 0.941 | 0.656 |
| PCF  | 0.879    | 0.583 | 0.863 | 0.513 | 1.000 | 0.968 | 0.801 |
| CCO  | 0.867    | 0.583 | 0.797 | 0.691 | 0.875 | 0.975 | 0.798 |
| PCO  | 0.879    | 0.792 | 0.887 | 0.770 | 1.000 | 0.985 | 0.886 |

FIG. 12

EP 4 787 380 A1

## INTERNATIONAL SEARCH REPORT

| International application No. |
| --- |
| **PCT/JP2024/033802** |

### A. CLASSIFICATION OF SUBJECT MATTER

*G16B 40/20*(2019.01)i

FI: G16B40/20

According to International Patent Classification (IPC) or to both national classification and IPC

### B. FIELDS SEARCHED

Minimum documentation searched (classification system followed by classification symbols)

G16B 5/00 - 99/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | US 2021/0381050 A1 (ADAPTIVE BIOTECHNOLOGIES CORPORATION) 09 December 2021 (2021-12-09)<br>entire text, all drawings | 1-17 |
| A | JP 2005-522750 A (ENTELOS, INC.) 28 July 2005 (2005-07-28)<br>entire text, all drawings | 1-17 |
| A | JP 2016-5469 A (FRED HUTCHINSON CANCER RESEARCH CENTER) 14 January 2016 (2016-01-14)<br>entire text, all drawings | 1-17 |

☐ Further documents are listed in the continuation of Box C.　　☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **25 November 2024** | **03 December 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/033802**

| Patent document cited in search report | | | Publication date (day/month/year) | Patent family member(s) | | | Publication date (day/month/year) |
|---|---|---|---|---|---|---|---|
| US | 2021/0381050 | A1 | 09 December 2021 | WO | 2016/138122 | A1 | |
| | | | | EP | 3262196 | A1 | |
| JP | 2005-522750 | A | 28 July 2005 | US | 2003/0104475 | A1 | |
| | | | | WO | 2003/001891 | A2 | |
| | | | | EP | 1410305 | A1 | |
| JP | 2016-5469 | A | 14 January 2016 | US | 2010/0330571 | A1 | |
| | | | | WO | 2010/151416 | A1 | |
| | | | | EP | 2446052 | A1 | |
| | | | | CN | 102459643 | A | |

Form PCT/ISA/210 (patent family annex) (July 2022)

**EP 4 787 380 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012508011 W

**Non-patent literature cited in the description**

- **FU, L.** ; **B. NIU** ; **Z. ZHU** ; **S. WU** ; **W. LI**. CD-HIT: accelerated for clustering the next-generation sequencing data. *Bioinformatics*, 2012, vol. 28 (23), 3150-3152

**25**